# EUROPEAN PATENT SPECIFICATION

(11) **EP 3 811 899 B1**
(45) Date of publication and mention of the grant of the patent: **10.04.2024**
(21) Application number: 19812617.9
(22) Date of filing: 16.05.2019
(51) Int. Cl.: A61F 2/04, A61F 2/848

(54) **STENT FOR BYPASS BETWEEN HOLLOW ORGANS AND STENT DELIVERY SYSTEM INCLUDING STENT FOR BYPASS BETWEEN HOLLOW ORGANS**
STENT FÜR BYPASS ZWISCHEN HOHLORGANEN UND STENTEINFÜHRSYSTEM MIT STENT FÜR BYPASS ZWISCHEN HOHLORGANEN
ENDOPROTHÈSE POUR PONTAGE ENTRE DES ORGANES CREUX ET SYSTÈME DE POSE D'ENDOPROTHÈSE COMPRENANT UNE ENDOPROTHÈSE POUR UNE DÉRIVATION ENTRE DES ORGANES CREUX

(30) Priority: 31.05.2018 JP 2018105337
(43) Date of publication of application: 28.04.2021
(73) Proprietor: Zeon Corporation, Tokyo 100-8246 (JP)
(72) Inventor: GON, Chimyon, Tokyo 100-8246 (JP)
(74) Representative: Maiwald GmbH
(86) International application number: PCT/JP2019/019459
(87) International publication number: WO 2019/230413

(56) References cited:
- WO-A1-2015/060424
- WO-A1-2016/069274
- JP-A- 2015 066 221
- JP-A- 2015 066 221
- US-A1- 2002 151 913
- US-A1- 2012 283 813
- US-A1- 2012 303 132
- US-B2- 8 747 345

## Description

### TECHNICAL FIELD

The present invention relates to a hollow organs bypass stent and a stent delivery system transporting the stent to a predetermined position in the body.

### BACKGROUND ART

In recent years, there have been reports that endoscopic ultrasound-guided biliary drainage (EUS-BD) was performed in the cases of unresectable malignant biliary stricture or obstruction that require biliary drainage with, for example, the transduodenal papilla approach impossible. EUS-BD is a procedure in which an ultrasonic endoscope is inserted into the stomach or duodenum, the bile duct or gallbladder is punctured with a puncture needle from the stomach wall or duodenal wall during real-time ultrasound image observation, a guide wire is inserted into the bile duct or gallbladder, and a stent for bypass is inserted and placed so as to bridge the stomach wall or duodenal wall and the bile duct or gallbladder. This procedure enables bile duct drainage by stent implantation in the body.

Stents used for EUS-BD include a covered stent in which the surface of a metallic stent base material is covered with a cover material made of a polymer film. In addition, a covered stent that has a hooking portion for preventing migration or organ wall damage has been proposed as a covered stent particularly suitable for EUS-BD (see Patent Document 1).

### CITATION LIST

### PATENT DOCUMENT

Patent Document 1: JP 2015-66221 A
Prior art US 2012/283813 A1 discloses an implantable medical devices deliverable through the vascular system to a target site, and especially discloses an implantable medical device for tissue penetration in order to treat a diseased human being by means of a catheter technique. The medical device for canalization of a tissue comprises a radially expandable and crimpable or collapsible substantially tubular member that has a mesh of wires arranged in-between a rear end and a front end, arranged around an interior of the device. The tubular member has extensions, which are arranged towards the interior of the device in a first state of the device, and towards an exterior of the medical device in a second state of the device. During delivery the device turns outside in and digs into the tissue to create a channel therein, thus preventing or fixating debris or other matter to spread from the channel.
In document WO 2016/069274 A1 an intralumenal stent graft device with integrated anchor features is disclosed that are selectively deployable and removable.
In prior art US 2002/0151913 A1 connector structures are discloses for attaching elongated flexible tubular grafts to the body organ tubing of a patient. The connector structures are formed from wire. If desired, hooks may be provided on the ends of the wires. The wires may be curved to accommodate attachment of the graft to tubular body organ tubing.
US 8,747,345 B2 discloses a bypass provided by a bypass conduit disposed in a vessel which is collateral to an occluded vessel. The bypass conduit comprises a helical wire body having a plurality of connectors connecting adjacent turns of the helical body.
Prior at JP 2015 066221 A discloses a stent for bypass between luminal organs that bypass-connects a luminal organ (eg, gastrointestinal tract such as stomach or duodenum) and another luminal organ (eg, bile duct or gallbladder).

### SUMMARY OF THE INVENTION

### PROBLEM TO BE SOLVED BY THE INVENTION

However, digestive tracts such as the stomach can be greatly deformed, and thus the migration prevention function of a hollow organs bypass stent for bypass-connecting a hollow organ to another hollow organ needs to be further enhanced such that the stent withstands hollow organ deformation and bypass is maintained.

The invention has been made in view of such circumstances, and an object of the invention is to provide a hollow organs bypass stent having a migration prevention function and a stent delivery system including the stent.

### MEANS FOR SOLVING PROBLEM

The above object is achieved by a hollow organs bypass stent having the features of claim 1. Preferred embodiments are specified in the dependent claims.

The hollow organs bypass stent according to the invention is capable of effectively preventing migration by the main body hooking coming into contact with the inner wall of a hollow organ or the like and functioning as an anchor. In addition, since the main body hooking portion is provided on the outer periphery other of the main body portion than both end portions, a part of the main body portion is capable of staying in the hollow organ even in a case where the main body hooking portion is slightly deformed and the indwelling position of the main body portion is misaligned. Accordingly, it is possible to effectively prevent the problem of the end portion of the stent falling out of the hollow organ.

In addition, for example, the main body hooking portion may have a curved shape curving to the outside of the main body portion along a longitudinal direction from a base end portion connected to the outer periphery toward a tip portion as a free end.

In the main body hooking portion having the curved shape, the elastic force that is required to stretch the curved shape of the main body hooking portion acts against a force to move the stent from the indwelling position, and thus it is possible to effectively prevent the hollow organs bypass stent from migrating or falling out of the hollow organ.

In addition, for example, the main body hooking portion may include a plurality of the main body hooking portions and at least two of the plurality of main body hooking portions may be disposed at the same distance from a first end portion as one of the both end portions.

By the two or more main body hooking portions being disposed at the same distance from the first end portion, it becomes easy to bring the plurality of main body hooking portions into contact with the inner wall of the hollow organ and it is possible to effectively prevent the hollow organs bypass stent from migrating or falling out of the hollow organ.

In addition, for example, the stent may further include a plurality of first end hooking portions independent of each other without being interconnected in the circumferential direction, wherein each of the first end hooking portions has a curved shape curving to the outside of the main body portion along a longitudinal direction from a base end portion connected to the first end portion toward a tip portion as a free end.

The stent that has both the main body hooking portion and the first end hooking portion provided in the end portion of the main body portion is capable of effectively preventing migration by means of the hooking portions provided at a plurality of positions. In addition, for example, the first end hooking portion provided in the first end portion comes into contact with the inner wall of the hollow organ and prevents the stent from moving even in a case where the main body hooking portion of the indwelling stent enters the body cavity side from the inner wall of the hollow organ due to deformation of the hollow organ or the like, and thus it is possible to effectively prevent the stent from falling out of the hollow organ in two stages.

In addition, for example, the main body hooking portion and the first end hooking portions may be curved in the same direction.

The hollow organs bypass stent is capable of more effectively preventing the stent from falling out of the hollow organ to the body cavity side in two stages.

According to a configuration not covered by the invention, the main body hooking portion and the first end hooking portions may be curved in opposite directions.

According to said configuration not covered by the invention, the hollow organs bypass stent is capable of effectively preventing migration or effectively preventing the stent from falling out by being placed in the body such that, for example, the wall of the hollow organ is sandwiched from both the outside and the inside between the main body hooking portion and the first end hooking portion.

In addition, for example, the main body portion may have a frame portion made of a metal material and a covering portion made of a polymer covering the frame portion.

The stent in which the main body portion has the frame portion and the covering portion is preferably used in a case where drainage or the like is performed.

The stent delivery system according to the invention includes the hollow organs bypass stent according to any one of the above and a transport mechanism transporting the hollow organs bypass stent to a predetermined position in a body.

### BRIEF DESCRIPTION OF DRAWINGS

Figs. 1A and 1B are conceptual diagrams illustrating an example of the stent delivery system according to the invention;
Fig. 2 is an external view illustrating an example of the hollow organs bypass stent according to the invention;
Fig. 3 is a cross-sectional view of the main body portion of the hollow organs bypass stent taken along line III-III illustrated in Fig. 2;
Fig. 4 is a partial development view illustrating the frame portion of the hollow organs bypass stent illustrated in Fig. 2 and a main body hooking portion and a first end hooking portion formed integrally with the frame portion;
Fig. 5 is a partially enlarged view of the hollow organs bypass stent illustrated in Fig. 4;
Fig. 6 is an external view of the hollow organs bypass stent according to a second embodiment;
Fig. 7 is a partial development view illustrating the frame portion of the hollow organs bypass stent illustrated in Fig. 6 and a main body hooking portion and a first end hooking portion formed integrally with the frame portion;
Fig. 8 is an external view of the hollow organs bypass stent according to a third embodiment; and
Fig. 9 is an external view of the hollow organs bypass stent according to a fourth embodiment.

### MODE(S) FOR CARRYING OUT THE INVENTION

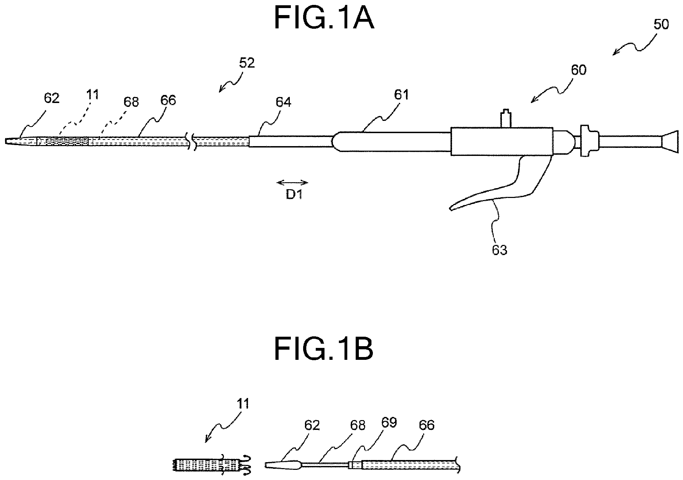

A hollow organs bypass stent and a stent delivery system including the stent of the invention will be described with reference to the embodiments illustrated in Figs. 1A-5, 8 and 9.

### First Embodiment

Figs. 1A and 1B are external views of a stent delivery system 50 according to a first embodiment of the invention. Fig. 1A illustrates the stent delivery system 50 in a first state where a stent 11 for bypass between hollow organs (hereinafter, sometimes simply referred to as "stent 11") is accommodated. Fig. 1B illustrates the distal end part of the stent delivery system 50 in a second state where the stent 11 is released. It should be noted that the direction in which the stent delivery system 50 extends from the proximal end where an operating portion 60 is disposed to the distal end where a distal tip 62 is disposed is an axial direction D1 in the description of the stent delivery system 50.

As illustrated in Figs. 1A and 1B, the stent delivery system 50 includes the stent 11 and a transport mechanism 52 transporting the stent 11 to a predetermined position in the body. The transport mechanism 52 has the operating portion 60, an outermost tube 64, an outer sheath 66, an inner shaft 68, the distal tip 62, and so on. The total length of the stent delivery system 50 is, for example, approximately 300 to 2,500 mm although the total length varies depending on, for example, the transport route or the indwelling position of the stent 11.

As illustrated in Fig. 1A, the stent 11 is accommodated near the distal end of the stent delivery system 50. A person who operates the stent delivery system 50 transports the stent 11 to the indwelling position in the body in the first state illustrated in Fig. 1A. Then, the operator releases the stent 11 and places the stent 11 at the predetermined position in the body by bringing the stent delivery system 50 into the second state illustrated in Fig. 1B.

As illustrated in Figs. 1A and 1B, the inner shaft 68 extends in the axial direction D1 from the operating portion 60 provided at the proximal end of the stent delivery system 50 to the distal tip 62 provided at the distal end of the stent delivery system 50. In the first state illustrated in Fig. 1A, the inner shaft 68 is accommodated in, for example, the outer sheath 66 and a housing 61 of the operating portion 60.

As illustrated in Fig. 1B, the inner shaft 68 has a small-diameter portion near the distal end and the small-diameter portion is smaller in outer diameter than the other part. In the second state where the outer sheath 66 has moved to the proximal end side, the small-diameter portion at the distal end is exposed. A guide wire lumen for passing a guide wire is formed in the inner shaft 68.

The distal tip 62 is provided at the distal end of the inner shaft 68. The distal tip 62 has a through hole communicating with the guide wire lumen of the inner shaft 68. The distal tip 62 is made of a resin or the like. The distal tip 62 has a rounded outer shape so as to be capable of preventing damage to the inner wall of a hollow organ in the event of contact with the inner wall.

The outer sheath 66 is relatively movable in the axial direction D1 with respect to the inner shaft 68 from the first state illustrated in Fig. 1A to the second state illustrated in Fig. 1B. The proximal end of the outer sheath 66 is accommodated in the housing 61 of the operating portion 60. The operating portion 60 moves the outer sheath 66 relative to the inner shaft 68 to the proximal end side, which is one side in the axial direction D1, in conjunction with the operation of an operation lever 63 attached to the housing 61. As a result, the stent delivery system 50 changes from the first state (Fig. 1A) where the outer sheath 66 covers the small-diameter portion of the inner shaft 68 to the second state (Fig. 1B) where the small-diameter portion of the inner shaft 68 and the stent 11 are exposed. It should be noted that the outermost tube 64 is provided near the operating portion 60 and further covers the outer periphery of the outer sheath 66. Although the movement of the outer sheath 66 by the operating portion 60 may be hindered when the operator directly grips the outer sheath 66, such a problem can be prevented by the outermost tube 64 covering the outer sheath 66.

The outermost tube 64, the outer sheath 66, and the inner shaft 68 of the transport mechanism 52 are made of, for example, a flexible resin tube. In addition, a metal wire rod may be embedded in the resin tube that is used for the outer sheath 66. It should be noted that a push ring 69 (see Fig. 1B) pushing the stent 11 in the axial direction D1 when the stent 11 is released may be provided on the proximal end side of the small-diameter portion in the inner shaft 68 and the push ring 69 may be made of a radiopaque material. In addition, although the materials of the operating portion 60 and the distal tip 62 in the transport mechanism 52 are not particularly limited, a molded or processed resin can be used as an example.

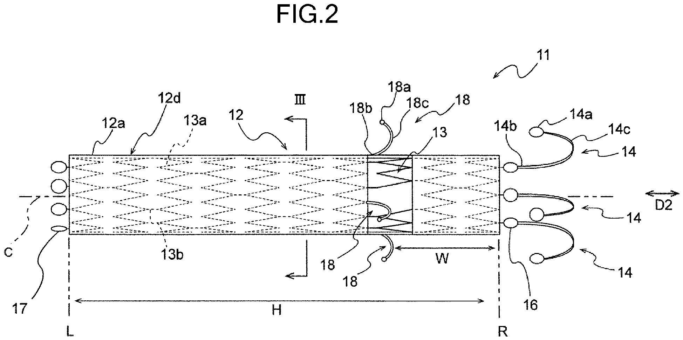

Fig. 2 is an external view of the stent 11 accommodated in the stent delivery system 50 and illustrates a state where the stent 11 is expanded in the radial direction. The stent 11 is a hollow organs bypass stent used for endoscopic ultrasound-guided biliary drainage (EUS-BD) as a hollow organs bypass stent for bypass-connecting a hollow organ to another hollow organ. In other words, the stent 11 is a stent for bypass that bypass-connects the stomach or duodenum to the bile duct or gallbladder. In addition, the stent 11 is a self-expanding covered stent that is contracted in the radial direction by elasticity when a compressive force is applied in the radial direction and expands in the radial direction when the compressive force is released. However, the hollow organs bypass stent of the invention is not limited thereto. The stent may be a balloon expansion-type covered stent. Further, the stent may be a stent such as a tube stent that does not expand in the radial direction.

As illustrated in Fig. 2, the stent 11 has an elongated and substantially tubular main body portion 12, a main body hooking portion 18 protruding from an outer periphery 12d of the main body portion 12 to the outside of the main body portion 12, a plurality of first end hooking portions 14 and a plurality of first end markers 16 provided in a first end portion (right end in Fig. 2) R, which is one of both end portions of the main body portion 12, and a plurality of second end markers 17 provided in a second end portion (left end in Fig. 2) L, which is the other end portion of the main body portion 12.

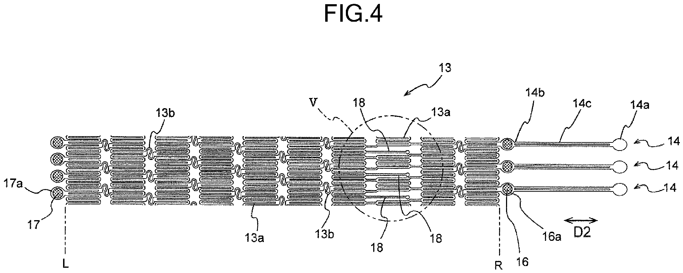

The main body portion 12 has a tubular frame portion 13 and a covering portion 12a covering the frame portion 13. Fig. 4 is a partial development view illustrating the frame portion 13 in the stent 11 illustrated in Fig. 2, the main body hooking portion 18 formed integrally with the frame portion 13, and the first end hooking portion 14. Fig. 4 illustrates a state where the stent 11 is maximally contracted in the radial direction. As illustrated in Fig. 4, the frame portion 13 is formed by a strut 13a and a bridge 13b, which are linear members made of a metal (or a resin). Here, the frame portion 13 may be formed by a wire rod being braided. Preferably, as illustrated in Fig. 4, the frame portion 13 is a so-called laser cut-type frame portion in which the strut 13a and the bridge 13b are formed by laser processing or the like being performed on a tube-shaped or pipe-shaped base material. When the frame portion 13 is a laser cut-type frame portion and expands in the radial direction, the shrinkage of the length of the frame portion 13 in an axial direction D2 is suppressed and the stent 11 can be easily placed at an intended position. The strut 13a and the bridge 13b constituting the frame portion 13 are capable of having a quadrangular or circular cross-sectional shape. It should be noted that the direction in which a central axis C of the tubular main body portion 12 extends is the axial direction D2 in the description of the stent 11 illustrated in Figs. 2 and 4. As illustrated in Fig. 1A, the stent 11 is accommodated in the transport mechanism 52 such that the axial direction D2 of the main body portion 12 matches the axial direction D1 of the stent delivery system 50.

As illustrated in the drawings including Fig. 2, the frame portion 13 has a plurality of the zigzag and annular struts 13a and the bridge 13b interconnecting the struts 13a that are adjacent to each other. The struts 13a are continuous in a triangular wave shape along the circumferential direction of the frame portion 13 and are connected in an annular shape. However, the struts 13a are connected in the circumferential direction, while meandering in an S shape, in a state where the frame portion 13 is maximally contracted (post-laser cut state) as illustrated in Fig. 4.

As illustrated in Figs. 2 and 4, the bridge 13b connects, in the axial direction D2, a part of the meandering curve part or the vertex of the triangular wave in the adjacent strut 13a. The tubular frame portion 13 is configured by the plurality of struts 13a being connected in this manner. Although the bridges 13b that interconnect the two adjacent struts 13a are disposed at substantially equal intervals, the positions of the bridges 13b that interconnect the two adjacent struts 13a are misaligned in the circumferential direction without being formed side by side in the axial direction D2.

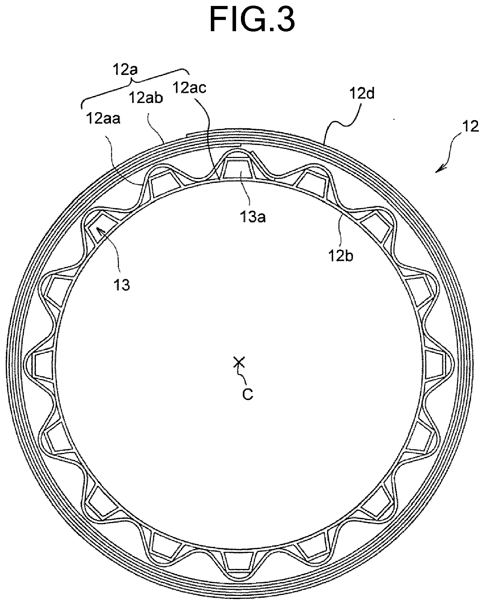

Fig. 3 is a cross-sectional view of the stent 11 illustrated in Fig. 2. As illustrated in Fig. 3, the frame portion 13 is covered with a coating film 12ac in the covering portion 12a and the coating film 12ac is spread so as to fill the space between the adjacent struts 13a and covers the outer periphery of the frame portion 13. The outer periphery of the frame portion 13 covered with the coating film 12ac is further covered with a first polymer film 12aa and a second polymer film 12ab in the covering portion 12a. However, the first polymer film 12aa and the second polymer film 12ab cover the outer periphery of the frame portion 13 only in a region other than the vicinity of the main body hooking portion 18 illustrated in Fig. 2.

A digestive fluid such as bile or the like is capable of flowing in the stent 11 by the space between the adjacent struts 13a being filled with the coating film 12ac. In addition, a stent inner peripheral surface 12b, which is the inner peripheral surface of the stent 11 (main body portion 12), can be smoothed by the frame portion 13 being covered with the coating film 12ac. By the stent inner peripheral surface 12b being smoothed, waste accumulation on the stent inner peripheral surface 12b is unlikely to occur in a case where the stent 11 is placed in the body. Infections and the like are prevented as a result.

Although a polymer such as an elastomer and a resin is used as the material of the coating film 12ac, a low-toxicity material that dissolves in an organic solvent is particularly preferable. Although a non-biodegradable polymer, a biodegradable polymer, or the like can be used as the polymer that can be used for the coating film 12ac, it is preferable to use a non-biodegradable polymer that is not easily decomposed in the living body and it is particularly preferable to use polyurethane, polytetrafluoroethylene (PTFE), or silicone resin among non-biodegradable polymers. It should be noted that the polymer constituting the coating film 12ac may be mixed with a drug such as an anticancer agent and an antithrombotic agent or an additive such as an anti-aging agent if necessary.

Although methods for forming the coating film 12ac on the frame portion 13 are not particularly limited, it is preferable to perform drying after immersing the frame portion 13 in a resin solution.

The covering portion 12a includes the second polymer film 12ab and the first polymer film 12aa covering the outer periphery of the frame portion 13. The first polymer film 12aa is disposed between the frame portion 13 and the second polymer film 12ab and covers the outer periphery of the frame portion 13.

The thickness of the first polymer film 12aa is preferably 4 to 20 µm as an average value of the entire first polymer film 12aa. If the first polymer film 12aa is too thick, the flexibility of the stent 11 may be insufficient. In addition, if the first polymer film 12aa is too thin, it may be impossible to protect the second polymer film 12ab from, for example, perforation by the frame portion 13.

The first polymer film 12aa is wound around the frame portion 13 so as to go around the outer periphery of the frame portion 13 by at least one turn and less than one and a half turns. By the number of windings of the first polymer film 12aa being 1 to 1.5, it is possible to prevent a shortage of flexibility for the main body portion 12 while protecting the second polymer film 12ab from, for example, perforation by the frame portion 13.

The second polymer film 12ab is wound around the first polymer film 12aa so as to go around the outer periphery of the first polymer film 12aa a plurality of times. By the second polymer film 12ab having a plurality of numbers of windings, it is possible to more reliably prevent the leakage of, for example, digested food or a digestive fluid such as bile passing through the stent 11. It should be noted that the covering portion 12a may be provided only at a part of the main body portion 12 although the covering portion 12a is provided in the entire main body portion 12 in the present embodiment illustrated in Fig. 2. For example, a region constituting the main body portion 12 only with the frame portion 13 may be present without the covering portion 12a being provided with a predetermined length along the axial direction D2 from the end portion (second end portion L) on the side of the main body portion 12 where the first end hooking portion 14 is not provided.

Although a polymer such as an elastomer and a resin is used as the material of the first polymer film 12aa and the second polymer film 12ab, a low-toxicity material that dissolves in an organic solvent is particularly preferable. In addition, from the viewpoint of protecting the second polymer film 12ab from perforation by the frame portion 13 or the like while ensuring the flexibility of the main body portion 12 of the stent 11, it is preferable to use a polymer higher in strength than the polymer that is the material of the second polymer film 12ab as the material of the first polymer film 12aa.

As illustrated in Fig. 2, a total length H of the main body portion 12 is determined in accordance with the distance between hollow organs to be bypass-connected, can be 10 mm to 200 mm, and is preferably 40 mm to 120 mm. In addition, an outer diameter D of the main body portion 12 that is expanded (with no external force applied) is determined in accordance with, for example, the type and size of a hollow organ to be bypass-connected, can be ϕ2 mm to ϕ20 mm, is preferably ϕ4 mm to ϕ15 mm, and is more preferably ϕ6 mm to ϕ10 mm. The outer diameter of the main body portion 12 that is contracted (accommodated in the stent delivery system) is approximately a fraction of the outer diameter of the main body portion 12 that is expanded.

As illustrated in Fig. 4, the wire diameter of the strut 13a constituting the frame portion 13 is preferably 0.05 to 1 mm. In addition, in a case where the strut 13a has a rectangular cross section, it is preferable that the length in the long side direction in the cross section of the strut 13a is 0.06 to 1 mm and the length in the short side direction in the cross section of the strut 13a is 0.05 to 0.9 mm. The outer diameter dimension of the frame portion 13 is substantially equal to the dimension of the main body portion 12 described above.

Resin or metal is used as the material of the frame portion 13. A resin that has appropriate hardness and elasticity can be used and a biocompatible resin is preferable as the resin that is used for the frame portion 13. Examples of the resin that is used as the material of the frame portion 13 include polyolefin, polyester, and fluororesin. In addition, specific examples of the polyester include polyethylene terephthalate and polybutylene terephthalate. In addition, specific examples of the fluororesin include polytetrafluoroethylene (PTFE) and ethylene-tetrafluoroethylene copolymer (ETFE).

Although examples of the metal that is used for the frame portion 13 include nickel-titanium (Ni-Ti) alloy, stainless steel, tantalum, titanium, cobalt-chrome alloy, and magnesium alloy, a superelastic alloy such as Ni-Ti alloy is preferable. Specific examples of the superelastic alloy that is used for the frame portion 13 include a Ni-Ti alloy containing 49 to 58 wt% of Ni. Also preferable as the material of the frame portion 13 is, for example, a Ni-Ti-X alloy (X being Co, Fe, Mn, Cr, V, Al, Nb, W, B, or the like) in which 0.01 to 10.0 wt% of the atoms in the Ni-Ti alloy are replaced with other atoms or a Ni-Ti-X alloy (X being Cu, Pb, or Zr) in which 0.01 to 30.0 wt% of the atoms in the Ni-Ti alloy are replaced with other atoms. The mechanical properties of these superelastic alloys are adjusted by cooling rate and/or final heat treatment conditions being selected.

The frame portion 13 can be molded by a tube-shaped or pipe-shaped base material being processed by laser processing using YAG laser or the like, electric discharge processing, chemical etching, cutting, or the like.

As illustrated in Figs. 2 and 4, the stent 11 has the plurality of first end markers 16 and the plurality of second end markers 17. The plurality of first end markers 16 have a disk shape as illustrated in Fig. 4 and are attached to the stent 11 by being fitted in the circular holes that are formed by circular frame-shaped first end marker frames 16a integrally provided in the first end portion (right end in Fig. 4) R, which is one of both end portions of the main body portion 12 (frame portion 13) of the stent 11. Likewise, the plurality of second end markers 17 have a disk shape as illustrated in Fig. 4 and are attached to the stent 11 by being fitted in the circular holes that are formed by circular frame-shaped second end marker frames 17a integrally provided in the second end portion (left end in Fig. 4) L, which is the other end portion of the main body portion 12 (frame portion 13) of the stent 11. The first end marker 16 and the second end marker 17 are made of, for example, an X-ray contrast material (radiopaque material). By confirming the positions of the first end marker 16 and the second end marker 17 under X-ray contrast with the stent 11 placed in the body, it is possible to grasp the indwelling position of the stent 11 (positions of both end portions of the main body portion 12).

In addition, as illustrated in Fig. 2, the stent 11 has a plurality of the main body hooking portions 18. The main body hooking portion 18 has an elongated outer shape extending from a base end portion 18b to a tip portion 18a. The main body hooking portion 18 protrudes to the outside of the main body portion 12 from the outer periphery 12d of the main body portion 12 other than the axial end portions R and L. The main body hooking portion 18 has a curved shape that curves to the outside of the main body portion 12 along the longitudinal direction from the base end portion 18b, which is connected to the outer periphery 12d, toward the tip portion 18a, which is a free end. However, in a state where the stent 11 is accommodated in the stent delivery system 50 as illustrated in Fig. 1A, the main body hooking portion 18 is in a state of being stretched in the axial direction D2 as illustrated in Fig. 4, which is a development view. Each main body hooking portion 18 is shaped so as to be curved to the outside of the main body portion 12 by its own elastic force when the stent 11 is released from the stent delivery system 50.

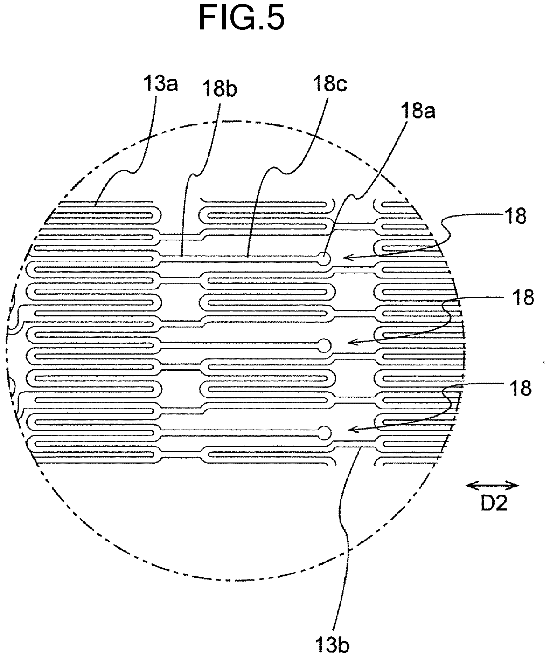

Fig. 4 illustrates three main body hooking portions 18. The three main body hooking portions 18 are integrated with the frame portion 13 made of a metal material and are molded from a pipe-shaped base material common with the frame portion 13. Fig. 5 is a partially enlarged view of Fig. 4 and illustrates the main body hooking portion 18 and the frame portion 13 around the main body hooking portion 18. As illustrated in Fig. 5, the base end portion 18b of the main body hooking portion 18 is connected to the strut 13a of the frame portion 13 adjacent to the base end portion 18b on the side opposite to the first end portion R side and protrudes from the strut 13a to the first end portion R side.

As illustrated in Fig. 4, at least two (three in Fig. 4) of the plurality of main body hooking portions 18 are disposed at the same distance W from the first end portion R, which is one of both end portions of the main body portion 12. Every main body hooking portion 18 included in the stent 11 may be disposed at the same distance W from the first end portion R as illustrated in Figs. 2 and 4. Alternatively, the plurality of main body hooking portions 18 may be disposed at different distances from the first end portion R. The distance W from the first end portion R to the main body hooking portion 18 is not particularly limited. Preferably, the distance W is 1/2 or less of the total length H of the main body portion 12. More preferably, the distance W is 1/4 or less of the total length H.

As illustrated in Fig. 2, the plurality of first end hooking portions 14 are provided in the first end portion (right end in Fig. 2) R, which is one of both end portions of the main body portion 12. As illustrated in Fig. 4, the first end hooking portion 14 is provided by being connected to the first end marker 16 connected to the first end portion R, has elasticity, and is formed by a linear, rod-shaped, or thin plate-shaped member extending in the longitudinal direction. Although the stent 11 has three first end hooking portions 14 as illustrated in Fig. 2, the number of the first end hooking portions 14 included in the stent 11 may be two, may be four or more, and is not particularly limited. In addition, the first end hooking portion 14 may be provided by being directly connected to the first end portion R of the main body portion 12.

The plurality of first end hooking portions 14 illustrated in Fig. 5 are independent of each other without being interconnected in the circumferential direction whereas the main body portion 12 is connected in the circumferential direction by the strut 13a and the covering portion 12a illustrated in Fig. 2 as illustrated in Figs. 2 and 4.

As illustrated in Fig. 2, the first end hooking portion 14 has a curved shape that curves to the outside of the main body portion 12 along the longitudinal direction from a base end portion 14b, which is connected to the first end portion R via the first end marker 16, to a tip portion 14a, which is a free end. However, in a state where the stent 11 is accommodated in the stent delivery system 50 as illustrated in Fig. 1A, the first end hooking portion 14 is in a state of being stretched in the axial direction D2 as illustrated in Fig. 4, which is a development view. Each first end hooking portion 14 is shaped so as to be curved to the outside of the main body portion 12 by its own elastic force when the stent 11 is released from the stent delivery system 50.

As illustrated in Figs. 4 and 5, in the present embodiment, the first end hooking portion 14 and the main body hooking portion 18 are formed integrally with the frame portion 13. Accordingly, the base end portions 14b and 18b of the first end hooking portion 14 and the main body hooking portion 18 are seamlessly connected to the frame portion 13. Although the cross-sectional shapes of the first end hooking portion 14 and the main body hooking portion 18 are not particularly limited, the shapes can be rectangular or circular and can be the same shape as the frame of the frame portion 13.

The first end hooking portion 14 and the main body hooking portion 18 may be integrally fixed by laser welding or the like after being formed independently of the frame portion 13 or may be cut out integrally with the frame portion 13 when the frame portion 13 is formed from a base material by laser processing or the like. In this case, the shapes (curved shapes) of the first end hooking portion 14 and the main body hooking portion 18 as illustrated in Fig. 2 can be molded by the first end hooking portion 14 and the main body hooking portion 18 being cut out into the shapes illustrated in Figs. 4 and 5 and then the cut first end hooking portion 14 and the cut main body hooking portion 18 being shaped as illustrated in Fig. 2.

As illustrated in Fig. 4, a slit extending in the longitudinal direction is formed in an intermediate portion 14c between the tip portion 14a and the base end portion 14b in the first end hooking portion 14. The base end portions 14b and 18b of the first end hooking portion 14 and the main body hooking portion 18 are respectively connected to the vertex portions of the triangular waves in the triangular wave-shaped struts 13a constituting the frame portion 13 (meandering curve parts in Fig. 5 illustrating a state where the stent 11 is maximally contracted in the radial direction). However, the main body hooking portion 18 provided between both end portions of the main body portion 12 may be connected to the bridge 13b.

The tip portions 14a and 18a of the first end hooking portion 14 and the main body hooking portion 18 are provided integrally with a substantially elliptical plate-shaped tip protecting portion having a smooth outer surface as illustrated in Figs. 4 and 5 so that damage to the inner wall of a hollow organ is prevented in the event of abutting against the inner wall or the like. It should be noted that the shape of the tip protecting portion may be, for example, a substantially disk or half-disk shape instead of the substantially elliptical plate shape insofar as the damage to the inner wall can be prevented when the tip portions 14a and 18a of the first end hooking portion 14 and the main body hooking portion 18 abut against the inner wall of the hollow organ or the like. In addition, the tip protecting portion may be formed by a substantially spherical member or the like being attached by post-processing without being provided integrally with the other part of the first end hooking portion 14 or the main body hooking portion 18.

As illustrated in Fig. 2, the intermediate portion 14c between the base end portion 14b and the tip portion 14a of the first end hooking portion 14 is smoothly curved and a part of the intermediate portion 14c on the base end portion 14b side is positioned outside the first end portion R (right side in Fig. 2) with regard to the axial direction D2 of the main body portion 12 and constitutes the end portion of the stent 11. In contrast, the tip portion 14a or the other part of the intermediate portion 14c in the first end hooking portion 14 on the tip portion 14a side is positioned inside the end portion of the stent 11 (left side in Fig. 2) with regard to the axial direction D2 of the main body portion 12.

As illustrated in Fig. 2, the intermediate portion 14c and an intermediate portion 18c of the first end hooking portion 14 and the main body hooking portion 18 are formed so as to be separated from the central axis C of the stent 11 toward the tip portion 14a and 18a sides. However, parts of the intermediate portions 14c and 18c of the first end hooking portion 14 and the main body hooking portion 18 on the tip portion 14a and 18a sides may be formed so as to approach the central axis C of the main body portion 12 toward the tip portion 14a side.

The curved shapes of the first end hooking portion 14 and the main body hooking portion 18 may have a uniform curvature as a whole. Alternatively, the curvature may change continuously or in stages toward the tip portions 14a and 18a. For example, parts of the intermediate portions 14c and 18c on the base end portion 14b and 18b sides may be larger or smaller in curvature than the other parts on the tip portion 14a and 18a sides. It should be noted that a single or a plurality of straight portions may be partially interposed in the curved shapes of the first end hooking portion 14 and the main body hooking portion 18. In addition, the curved shapes of the first end hooking portion 14 and the main body hooking portion 18 are not limited to a curve having one inflection point and may be a curve having two or more inflection points.

As illustrated in Fig. 2, the main body hooking portion 18 and the first end hooking portion 14 are curved in the same direction. In other words, each of the main body hooking portion 18 and the first end hooking portion 14 has a shape in which its part that starts to curve from the outer periphery 12d of the main body portion 12 curves outward from the central axis C when the part proceeds in the direction away from the middle of the main body portion 12 along the axial direction D2. The stent 11 is placed such that, for example, both the main body hooking portion 18 and the first end hooking portion 14 are positioned on the hollow organ side. As a result, it is possible to effectively prevent the problem of the stent 11 falling out of the hollow organ to the body cavity side in the two stages of the main body hooking portion 18 and the first end hooking portion 14.

In this embodiment, the number of the first end hooking portions 14 is three, the number of the main body hooking portions 18 is three, and the first end hooking portions 14 and the main body hooking portions 18 are substantially radially disposed at equal angular intervals (that is, 120 degrees) as for the circumferential disposition thereof. Alternatively, each of the numbers may be two or four or more. Although the first end hooking portions 14 and the main body hooking portions 18 may be disposed at equal angular intervals, the invention is not necessarily limited thereto and the disposition can be appropriately selected in accordance with, for example, the type or shape of a hollow organ to be applied. In addition, although the plurality of first end hooking portions 14 (three in the present embodiment) have the same configurations (curved shape, length, and so on) without exception in the present embodiment, one or more of the plurality of first end hooking portions 14 may be different in configuration (curved shape, length, and so on). Likewise, the plurality of main body hooking portions 18 (three in the present embodiment) may have the same configurations without exception or one or more of the plurality of main body hooking portions 18 may be different in configuration.

As illustrated in Figs. 2 and 4, the second end portion (left end in Figs. 2 and 4) L, which is the other end portion of the main body portion 12, is not provided with a hooking portion unlike the first end portion R. The stent 11 may be provided with a hooking portion only in one end portion of the main body portion 12 as illustrated in Figs. 2 and 4. Alternatively, hooking portions may be provided in both end portions of the main body portion 12 as in the stent according to a fourth embodiment to be described later.

As described above, the stent 11 has the main body hooking portion 18 provided on the outer periphery 12d other than the end portions R and L of the main body portion 12. Accordingly, the first end portion R is capable of staying in the hollow organ even in a case where the indwelling position of the main body portion 12 is slightly misaligned and it is possible to effectively prevent the problem of the end portion of the stent 11 falling out of the hollow organ. In addition, in the stent 11, the first end hooking portion 14 in the first end portion R is provided in addition to the main body hooking portion 18. Accordingly, the main body hooking portion 18 and the first end hooking portion 14 are capable of preventing the problem of the stent 11 falling out of the hollow organ in multiple stages even in a case where a force acts on the stent 11 to pull the first end portion R into the body cavity side.

### Second Embodiment (not covered by the present invention)

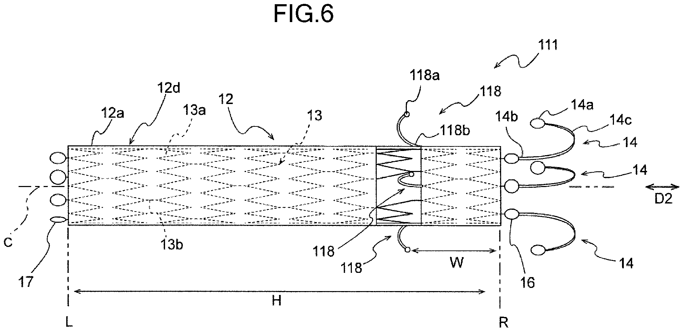

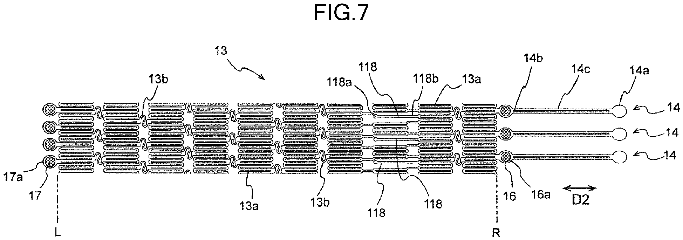

Fig. 6 is an external view of a stent 111 according to a second embodiment not covered by the invention. Fig. 7 is a partial development view illustrating the frame portion 13 included in the stent 111 illustrated in Fig. 6 and the first end hooking portion and a main body hooking portion formed integrally with the frame portion 13. The stent 111 according to the second embodiment is the same as the stent 11 according to the first embodiment except that a main body hooking portion 118 is different in shape from the main body hooking portion 18 illustrated in Figs. 2 and 4. Accordingly, in the description of the stent 111, the same reference numerals are given to points common to the stent 111 and the stent 11 and description thereof are omitted.

As illustrated in Fig. 6, the stent 111 has a plurality of the main body hooking portions 118 (three in the embodiment). The main body hooking portion 118 has an elongated outer shape extending from a base end portion 118b to a tip portion 118a. The main body hooking portion 118 has a curved shape that curves to the outside of the main body portion 12 along the longitudinal direction from the base end portion 118b, which is connected to the outer periphery 12d, toward the tip portion 118a, which is a free end. The distance W from the first end portion R where the first end hooking portion 14 is provided to the main body hooking portion 18 is (1/2) or less of the total length H of the main body portion 12 as in the case of the stent 11 illustrated in Fig. 2.

As illustrated in Fig. 6, the main body hooking portion 118 and the first end hooking portion 14 are curved in opposite directions. In other words, the first end hooking portion 14 has a shape in which its part that starts to curve from the outer periphery 12d of the main body portion 12 curves outward from the central axis C when the part proceeds in the direction away from the middle of the main body portion 12 along the axial direction D2. In contrast, the main body hooking portion 118 has a shape in which its part that starts to curve from the outer periphery 12d of the main body portion 12 curves outward from the central axis C when the part proceeds toward the middle of the main body portion 12 along the axial direction D2.

As illustrated in Fig. 7, the base end portion 118b of the main body hooking portion 118 is connected to the strut 13a of the frame portion 13 adjacent to the base end portion 118b on the first end portion R side and protrudes from the strut 13a to the side opposite to the first end portion R side.

It is conceivable that the stent 111 according to the second embodiment is used by being placed in the body such that, for example, the wall of the hollow organ is sandwiched from both the outside and the inside by the main body hooking portion 118 and the first end hooking portion 14. In the stent 111, the main body hooking portion 118 and the first end hooking portion 14 work in cooperation with each other and both the migration in which the stent 111 moves to the body cavity side and the migration in which the stent 111 moves to the hollow organ side can be effectively prevented.

### Third Embodiment

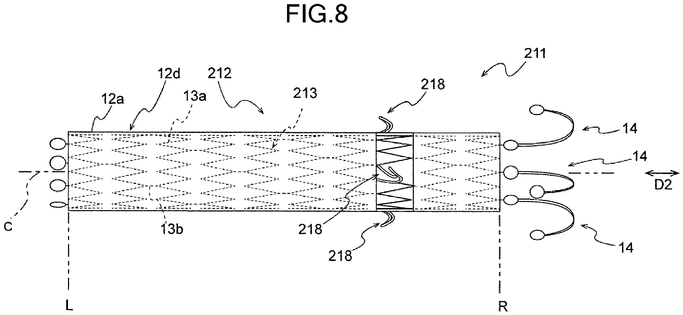

Fig. 8 is an external view of a stent 211 according to a third embodiment of the invention. The stent 211 according to the third embodiment is the same as the stent 11 according to the first embodiment except that a main body hooking portion 218 and a frame portion 213 around the main body hooking portion 218 are different in shape from the main body hooking portion 18 and the frame portion 13 illustrated in Fig. 2. Accordingly, in the description of the stent 211, the same reference numerals are given to points common to the stent 211 and the stent 11 and description thereof are omitted.

The main body hooking portion 218 of the stent 211 is formed by a part of the strut 13a included in the frame portion 213 being bent to the outside of a main body portion 212 and exposed to the outside of the covering portion 12a.

The stent 211 illustrated in Fig. 8 is excellent in productivity because the stent 211 is capable of being common in processing shape with another frame portion lacking the main body hooking portion 218 when the frame portion 213 and the main body hooking portion 218 are created by laser processing or the like. In addition, the stent 211 according to the third embodiment has the same effect as the stent 11 according to the first embodiment. It should be noted that the shape of the main body hooking portion of the stent according to the invention is not limited to the shape described above and another shape may be adopted insofar as the main body hooking portion protrudes from the outer periphery 12d.

### Fourth Embodiment

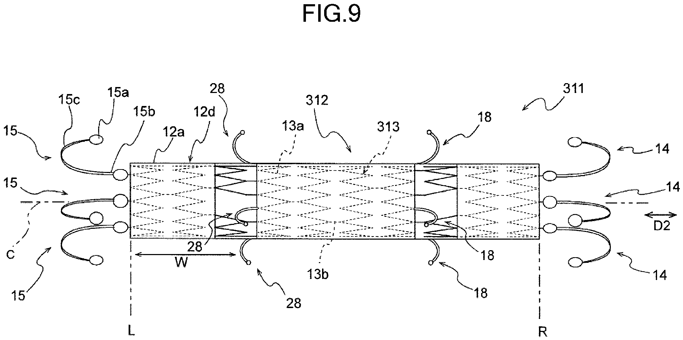

Fig. 9 is an external view of a stent 311 according to the fourth embodiment of the invention. The stent 311 according to the fourth embodiment is different from the stent 11 according to the first embodiment in that, for example, a plurality of second end hooking portions 15 are provided in the second end portion L and a main body hooking portion 28 is provided closer to the second end portion L side than the middle of a main body portion 312 in the axial direction D2. However, the main body hooking portion 18 and the first end hooking portion 14 are common to the stent 11 according to the first embodiment and the stent 311. In the description of the stent 311, the same reference numerals are given to points common to the stent 311 and the stent 11 and description thereof are omitted.

The position of the main body hooking portion 28 of the stent 311 with regard to the axial direction D2 and the direction in which the main body hooking portion 28 of the stent 311 curves are different from those of the main body hooking portion 18. In other aspects, the main body hooking portion 28 of the stent 311 is the same as the main body hooking portion 18 provided closer to the first end portion R side than the middle of the main body portion 312 in the axial direction D2. In other words, the main body hooking portion 28 is provided at a position at the distance W from the second end portion L and protrudes outward from the outer periphery 12d of the main body portion 312. The distance W from the second end portion L to the main body hooking portion 28 is equal to the distance W from the first end portion R to the main body hooking portion 18. The cross section of the stent 311 is the same as the cross section of the stent 11 illustrated in Fig. 3 with regard to a region other than the vicinity of the main body hooking portions 18 and 28. In addition, in the cross section of the stent 311, the first polymer film 12aa and the second polymer film 12ab illustrated in Fig. 3 cover the outer periphery of the frame portion 13 only in the region other than the vicinity of the main body hooking portions 18 and 28.

As illustrated in Fig. 9, the second end hooking portion 15 has a curved shape that curves to the outside of the main body portion 312 along the longitudinal direction from a base end portion 15b, which is connected to the second end portion L, toward a tip portion 15a, which is a free end. The plurality of second end hooking portions 15 are independent of each other without being interconnected in the circumferential direction. The plurality of second end hooking portions 15 have the same shape and structure as the first end hooking portion 14 described above except that the plurality of second end hooking portions 15 are provided not in the first end portion R but in the second end portion L. Accordingly, the detailed structure of the second end hooking portion 15 will not be described.

As illustrated in Fig. 9, the main body hooking portion 28 and the second end hooking portion 15 are curved in the same direction. In other words, each of the main body hooking portion 28 and the second end hooking portion 15 has a shape in which its part that starts to curve from the outer periphery 12d of the main body portion 312 curves outward from the central axis C when the part proceeds in the direction away from the middle of the main body portion 12 along the axial direction D2.

The stent 311 according to the fourth embodiment is provided with the first end hooking portion 14, the second end hooking portion 15, and the main body hooking portions 18 and 28 on both sides of the main body portion 312. Accordingly, the hooking portions 14, 15, 18, and 28 are capable of preventing the end portions R and L of the main body portion 312 from coming out of the hollow organ in multiple stages.

### EXPLANATIONS OF LETTERS OR NUMERALS

- 11, 111, 211, 311: HOLLOW ORGANS BYPASS STENT
- 12, 212, 312: MAIN BODY PORTION
- 12a: COVERING PORTION
- 12aa: FIRST POLYMER FILM
- 12ab: SECOND POLYMER FILM
- 12b: STENT INNER PERIPHERAL SURFACE
- 12ac: COATING FILM
- 12d: OUTER PERIPHERY
- R: FIRST END PORTION
- L: SECOND END PORTION
- 13, 213, 313: FRAME PORTION
- 13a: STRUT
- 13b: BRIDGE
- 14: FIRST END HOOKING PORTION
- 15: SECOND END HOOKING PORTION
- 16: FIRST END MARKER
- 16a: FIRST END MARKER FRAME
- 17: SECOND END MARKER
- 17a: SECOND END MARKER FRAME
- 18, 28, 118, 218: MAIN BODY HOOKING PORTION
- 14a, 15a, 18a, 118a: TIP PORTION
- 14b, 15b, 18b, 118b: BASE END PORTION
- 14c, 18c: INTERMEDIATE PORTION
- 50: STENT DELIVERY SYSTEM
- 52: TRANSPORT MECHANISM
- 60: OPERATING PORTION
- 61: HOUSING
- 62: DISTAL TIP
- 63: OPERATION LEVER
- 64: OUTERMOST TUBE
- 66: OUTER SHEATH
- 68: INNER SHAFT
- 69: PUSH RING
- D1: AXIAL DIRECTION OF DELIVERY SYSTEM
- D2: AXIAL DIRECTION OF MAIN BODY PORTION
- C: CENTRAL AXIS

## Claims

1. A hollow organs bypass stent (11) for bypass-connecting a stomach or a duodenum to a bile duct or a gallbladder, comprising:
an elongated and tubular main body portion (12); and
a main body hooking portion (18) protruding to an outside of the main body portion (12) from an outer periphery of the main body portion (12) other than both axial end portions,
**characterized in that** the stent is prevented from migrating by the main body hooking portion coming into contact with the inner wall of a hollow organ and functioning as an anchor.

2. The hollow organs bypass stent (11) according to claim 1, wherein the main body hooking portion (18) has a curved shape curving radially outwards from a central axis of the main body portion (12) along a longitudinal direction from a proximal end portion (18b) connected to the outer periphery toward a tip portion (18a) as a free end.

3. The hollow organs bypass stent (11) according to claim 1 or 2, wherein the main body hooking portion (18) comprises a plurality of main body hooking portions (18), and at least two of the plurality of main body hooking portions (18) are disposed at the same distance from a first end portion (R) which is one of the both end portions (R, L).

4. The hollow organs bypass stent (11) according to any one of claims 1 to 3, further comprising a plurality of first end hooking portions (14) independent of each other without being interconnected in the circumferential direction, wherein each of the first end hooking portions (14) has a curved shape curving radially outwards from a central axis of the main body portion (12) along a longitudinal direction from a proximal end portion (14b) connected to a first end portion (R) which is one of the both end portions (R, L) toward a tip portion (14a) as a free end.

5. The hollow organs bypass stent (11) according to claim 4, wherein the main body hooking portion (18) and the first end hooking portions (14) are curved in the same direction.

6. The hollow organs bypass stent (11) according to any one of claims 1 to 5, wherein the main body portion (12) has a frame portion (13) made of a metal material and a covering portion (12a) made of a polymer covering the frame portion (13).

7. A stent delivery system (50) comprising:
the hollow organs bypass stent (11, 111) according to any one of claims 1 to 6; and
a transport mechanism (52) transporting the hollow organs bypass stent (11, 111) to a predetermined position in a body.

## Patentansprüche

1. Ein Hohlorgan-Bypass-Stent (11) zum Bypass-Verbinden eines Magens oder eines Zwölffingerdarms mit einem Gallengang oder einer Gallenblase, umfassend:
einen länglichen und röhrenförmigen Hauptkörperabschnitt (12); und
einen Hauptkörper-Hakenabschnitt (18), der zu einer Außenseite des Hauptkörperabschnitts (12) von einem Außenumfang des Hauptkörperabschnitts (12) außer den beiden axialen Endabschnitten vorsteht,
**dadurch gekennzeichnet, dass** der Stent am Wandern gehindert wird, indem der Hauptkörper- Hakenabschnitt mit der Innenwand eines Hohlorgans in Kontakt kommt und als Anker fungiert.

2. Der Hohlorgan-Bypass-Stent (11) nach Anspruch 1, wobei der Hauptkörper-Hakenabschnitt (18) eine gekrümmte Form aufweist, die sich von einer zentralen Achse des Hauptkörperabschnitts (12) entlang einer Längsrichtung von einem proximalen Endabschnitt (18b), der mit dem äußeren Umfang verbunden ist, zu einem Spitzenabschnitt (18a) als freies Ende radial nach außen krümmt.

3. Der Hohlorgan-Bypass-Stent (11) nach Anspruch 1 oder 2, wobei der Hauptkörper-Hakenabschnitt (18) eine Vielzahl von Hauptkörper-Hakenabschnitten (18) umfasst, und mindestens zwei der mehreren Hauptkörper-Hakenabschnitte (18) im gleichen Abstand von einem ersten Endabschnitt (R), der einer der beiden Endabschnitte (R, L) ist, angeordnet sind.

4. Der Hohlorgan-Bypass-Stent (11) nach einem der Ansprüche 1 bis 3, weiterhin umfassend eine Mehrzahl von ersten Endhakenabschnitten (14), die unabhängig voneinander sind, ohne in der Umfangsrichtung miteinander verbunden zu sein, wobei jeder der ersten Endhakenabschnitte (14) eine gekrümmte Form hat, die sich von einer zentralen Achse des Hauptkörperabschnitts (12) entlang einer Längsrichtung von einem proximalen Endabschnitt (14b), der mit einem ersten Endabschnitt (R) verbunden ist, der einer der beiden Endabschnitte (R, L) ist, zu einem Spitzenabschnitt (14a) als ein freies Ende radial nach außen krümmt.

5. Der Hohlorgan-Bypass-Stent (11) nach Anspruch 4, wobei der Hauptkörper-Hakenabschnitt (18) und die ersten Endhakenabschnitte (14) in die gleiche Richtung gekrümmt sind.

6. Der Hohlorgan-Bypass-Stent (11) nach einem der Ansprüche 1 bis 5,
wobei der Hauptkörperabschnitt (12) einen Rahmenabschnitt (13) aus einem Metallmaterial und einen Abdeckungsabschnitt (12a) aus einem Polymer aufweist, der den Rahmenabschnitt (13) abdeckt.

7. Ein Stent-Einführsystem (50), umfassend:
den Hohlorgane-Bypass-Stent (11, 111) nach einem der Ansprüche 1 bis 6; und
einen Transportmechanismus (52), der den Hohlorgan-Bypass-Stent (11, 111) zu einer vorbestimmten Position in einem Körper transportiert.

## Revendications

1. Endoprothèse de dérivation (11) pour organes creux afin de raccorder, par dérivation, un estomac ou un duodénum à un conduit cholédoque ou une vésicule biliaire, comprenant :
une partie de corps principal allongée et tubulaire (12) ; et
une partie d'accrochage de corps principal (18) faisant saillie à un extérieur de la partie de corps principal (12) à partir d'une périphérie externe de la partie de corps principal (12) différente des deux parties d'extrémité axiales,
**caractérisée en ce que** l'endoprothèse ne peut pas migrer grâce à la partie d'accrochage de corps principal venant en contact avec la paroi interne d'un organe creux et servant d'ancrage.

2. Endoprothèse de dérivation (11) pour organes creux selon la revendication 1, dans laquelle la partie d'accrochage de corps principal (18) a une forme courbée se courbant radialement vers l'extérieur à partir d'un axe central de la partie de corps principal (12) le long d'une direction longitudinale à partir d'une partie d'extrémité proximale (18b) raccordée à la périphérie externe vers une partie de pointe (18a) en tant qu'extrémité libre.

3. Endoprothèse de dérivation (11) pour organes creux selon la revendication 1 ou 2, dans laquelle la partie d'accrochage de corps principal (18) comprend une pluralité de parties d'accrochage de corps principal (18), et au moins deux de la pluralité de parties d'accrochage de corps principal (18) sont disposées à la même distance d'une première partie d'extrémité (R) qui est l'une des deux parties d'extrémité (R, L).

4. Endoprothèse de dérivation (11) pour organes creux selon l'une quelconque des revendications 1 à 3, comprenant en outre une pluralité de premières parties d'accrochage d'extrémité (14) indépendantes les unes des autres sans être interconnectées dans la direction circonférentielle, dans laquelle chacune des premières parties d'accrochage d'extrémité (14) a une forme courbée se courbant radialement vers l'extérieur à partir d'un axe central de la partie de corps principal (12) le long d'une direction longitudinale à partir d'une partie d'extrémité proximale (14b) raccordée à une première partie d'extrémité (R) qui est l'une des deux parties d'extrémité (R, L) vers une partie de pointe (14a) en tant qu'extrémité libre.

5. Endoprothèse de dérivation (11) pour organes creux selon la revendication 4, dans laquelle la partie d'accrochage de corps principal (18) et les premières parties d'accrochage d'extrémité (14) sont courbées dans la même direction.

6. Endoprothèse de dérivation (11) pour organes creux selon l'une quelconque des revendications 1 à 5, dans laquelle la partie de corps principal (12) a une partie de bâti (13) réalisée à partir d'un matériau métallique et une partie de revêtement (12a) réalisée à partir d'un polymère recouvrant la partie de bâti (13).

7. Système de pose d'endoprothèse (50) comprenant :
l'endoprothèse de dérivation (11, 111) pour organes creux selon l'une quelconque des revendications 1 à 6 ; et
un mécanisme de transport (52) transportant l'endoprothèse de dérivation (11, 111) pour organes creux dans une position prédéterminée dans un corps.
